# EUROPÄISCHE PATENTSCHRIFT

(11) **EP 1 786 389 B1**
(45) Veröffentlichungstag und Bekanntmachung des Hinweises auf die Patenterteilung: **24.07.2013**
(21) Anmeldenummer: 05764389.2
(22) Anmeldetag: 04.08.2005
(51) Int. Cl.: A61K 8/18, A61Q 5/06, A61K 8/39, A61K 8/86, A61K 8/19, A61K 8/46

(54) **HAUTSCHONENDE WELLMITTEL**
SKIN-PROTECTING WAVING AGENT
PRODUITS POUR PERMANENTE INOFFENSIFS POUR LA PEAU

(30) Priorität: 08.09.2004 DE 102004043368
(43) Veröffentlichungstag der Anmeldung: 23.05.2007
(73) Patentinhaber: Henkel AG & Co. KGaA, 40589 Düsseldorf (DE)
(72) Erfinder: LUDWIG, Meike, 22085 Hamburg (DE); MÜLLER, Burkhard, 21075 Hamburg (DE)
(86) Internationale Anmeldenummer: PCT/EP2005/008439
(87) Internationale Veröffentlichungsnummer: WO 2006/027059

(56) Entgegenhaltungen:
- DE-A- 19 816 662
- D. WILLIAMS ET AL.: "Chemistry and technology of the cosmetics and toiletries industry, ed.2" 1996, BLACKIE ACADEMIC , GB , XP002348939 Seite 88
- M.L. SCHLOSSMANN ED.: "The Chemistry and Manufacture of Cosmetics: Formulating. Vol.2, ed.3" 2000, ALLURED PUB. , US , XP002348940 Seite 524 - Seite 525

## Beschreibung

Die Erfindung betrifft die Verwendung von Fettalkoholen mit 10 bis 14 Kohlenstoffatomen, die mit 8 bis 12 Mol Ethylenoxid ethoxyliert sind, zur Reduzierung der Hautreizung durch Zusammensetzungen, enthaltend mindestens eine schwefelhaltige, keratinreduzierende Substanz. Weiterhin werden Mittel zur dauerhaften Verformung von Keratinfasern, insbesondere von menschlichen Haaren, beschrieben, welche neben mindestens einer schwefelhaltigen, keratinreduzierenden Substanz mindestens eine Verbindung enthalten, die ausgewählt wird aus mit 8 bis 12 Mol Ethylenoxid ethoxylierten Fettalkoholen mit 10 bis 14 Kohlenstoffatomen, sowie ein Verfahren zur dauerhaften Verformung keratinhaltiger Fasern, in welchem das Mittel Anwendung findet.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung der keratinreduzierenden Substanz ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist oftmals ein Verspröden und Stumpfwerden der Haare. Desweiteren kann, insbesondere bei Personen mit sensibler Haut, eine Reizung der Kopfhaut beobachtet werden. Für diese Kopfhautreizung zeichnen sich die schwefelhaltigen, keratinreduzierenden Substanzen verantwortlich.

Es hat in der Vergangenheit nicht an Versuchen gefehlt, Wellmittel zu formulieren, welche eine geringere Hautreizung verursachen.

In der Druckschrift DE-C2-199 02 246 werden Wellmittel beschrieben, welche eine zeitverzögerte Senkung des pH-Werts erfahren. Auf diesem Wege soll die Haarstruktur während der Anwendungsdauer des Wellmittels geschützt werden. Es hat sich aber herausgestellt, dass diese Wellmittel noch immer die Kopfhaut reizen. Durch die allmähliche pH-Wert Senkung während der Anwendung erhöht sich die Einwirkzeit der Wellmittel und somit auch die Kontaktzeiten der Wellmittel mit der Kopfhaut. Um eine Kopfhautreizung zu verhindern ist jedoch die Anwendungsdauer des Wellmittels möglichst kurz zu halten.
D. Williams und al. offenbart zwei Zusammensetzungen für die dauerhafte Verformung von menschlichen Haaren die Laureth-12 und eine Thioglykolsäure enthalten.
M.L. Schlossmann und al. offenbart eine Zusammensetzung für die Haare mit Glycerylthioglykolate.
DE19816662 offenbart ein Haarverformungsmittel die eine Zusammensetzung öl-in-Wasser enthält.
C. Mc Cord und al. offenbart das Problem von Hautreizung durch Schwefelhaltige Substanzen.
JP01258616 offenbart Haarentternungsmitteln mit Thioglykolsäure.
CA1315204, US6024735 und US5985275 offenbaren Zusammensetzung die Laureth-10 enthalten.
WO96/19228 offenbart Zusammensetzungen die eine reduzierte Hautreizung zeigen.

Es bestand somit die Aufgabe, ein Mittel für die dauerhafte Verformung von keratinhaltigen Fasern bereitzustellen, nach dessen Anwendung die genannten unerwünschten Nebenwirkungen der keratinreduzierenden Substanzen reduziert oder ganz ausgeschlossen werden. Gleichzeitig soll eine sehr gute Wellwirkung erreicht werden.

Überraschenderweise wurde gefunden, dass bestimmte ethoxylierte Fettalkohole die Hautreizung der keratinreduzierenden Substanzen reduzieren.

Ein erster Gegenstand der Erfindung ist die Verwendung von mit 10 Einheiten Ethylenoxid ethoxylierte

Cocosfettalkoholen aus überwiegend (C₁₂ - bis C₁₄)- Fettalkoholen (I) zur Verminderung der Hautreizung von schwefelhaltigen, keratinreduzierenden Substanzen.

Die Verbindungen (I) sind insbesondere wirksam, wenn Sie mit schwefelhaltigen, keratinreduzierenden Substanzen der Mercaptane mit mindestens einer Gruppe -SH oder Sulfit-Verbindungen, wie z.B. Sulfite, Hydrogensulfite oder Disulfite, verwendet werden.

Beispiele für schwefelhaltige, keratinreduzierende Substanzen der Disulfite sind Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für schwefelhaltige, keratinreduzierende Substanzen der Hydrogensulfite sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für schwefelhaltige, keratinreduzierende Substanzen der Sulfite sind Sulfite als Alkali-, Ammonium- oder Alkanolammonium-Salz auf Basis eines C₂-C₄-Mono-, Di- oder Trialkanolamins. Ammoniumsulfit ist dabei bevorzugt.

Bevorzugte schwefelhaltige, keratinreduzierende Substanzen sind Cysteamin, Cystein, Bunte Salze und Salze der schwefligen Säure, Thioglykolsäure, Thiomilchsäure, Thioäpfelsäure, Mercaptoethansulfonsäure sowie den Salzen und Estern der vorgenannten Thiosäuren. Besonders wirksam sind die Verbindungen in Kombination mit Mercaptogruppenhaltigen keratinreduzierenden Substanzen.

Die (C₁₂- bis C₁₄)-Alkylgruppe sind bevorzugt linear.

Besonders bevorzugte Verbindungen (I) sind die dem Fachmann unter den folgenden INCI-Bezeichnungen bekannten Verbindungen Laureth-8 (z.B. Empilan^{®} KB 8 der Fa. Albright & Wilson UK Ltd.), Laureth-10 (z.B. Empilan^{®} KB 10 der Fa. Albright & Wilson UK Ltd.), Laureth-12 (z.B. Empilan^{®} KB 12 der Fa. Albright & Wilson UK Ltd.), Coceth-8, Coceth-10 (z.B. Genapol^{®} C 100 der Fa. Clariant) und Myreth-10 (z.B. der Mischung Isoxal^{®} 11 der Fa. Vevy Europe SpA (INCI-Bezeichnung: Ceteth-10, Steareth-10, Myreth-10, Laureth-10)).

Die Verbindungen (I) sind mit 10 Einheiten Ethylenoxid ethoxylierte Cocosfettalkohole aus überwiegend (C₁₂- bis C₁₄)-Fettalkoholen. Diese sind beispielsweise unter dem Handelsnamen Genapol^{®} C 100 (INCI-Bezeichnung: Coceth-10; Firma Clariant) mit einem HLB-Wert von 14 erhältlich.

Ein zweiter Gegenstand der Erfindung sind Mittel für die dauerhafte Verformung keratinhaltiger Fasern, insbesondere menschlicher Haare, welche einen konstanten pH-Wert aufweisen und
(a) mindestens eine schwefelhaltige, keratinreduzierende Substanz sowie
(b) mit 10 Einheiten Ethylenoxid ethoxylierte Cocosfettalkoholen aus überwiegend (C₁₂- bis C₁₄)- Fettalkoholen (I).

Die erfindungsgemäßen Wellmittel enthalten bevorzugt die als schwefelhaltige, keratinreduzierende Substanzen bekannten Mercaptane mit mindestens einer Gruppe -SH oder Sulfit-Verbindungen, wie z.B. Sulfite, Hydrogensulfite oder Disulfite. Beispiele für keratinreduzierende Substanzen der Disulfite sind Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Substanzen der Hydrogensulfite sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Substanzen der Sulfite sind Sulfite als Alkali-, Ammonium- oder Alkanolammonium-Salz auf Basis eines C₂-C₄-Mono-, Di- oder Trialkanolamins. Ammoniumsulfit ist dabei bevorzugt.

Bevorzugte schwefelhaltige, keratinreduzierende Substanzen sind aus der Gruppe ausgewählt, die gebildet wird aus Cysteamin, Cystein, Bunte Salze und Salze der schwefligen Säure, Thioglykolsäure, Thiomilchsäure, Thioäpfelsäure, Mercaptoethansulfonsäure sowie den Salzen und Estern der vorgenannten Thiosäuren. Bevorzugt geeignet sind die Alkali- oder Ammoniumsalze der Thioglykolsäure und/oder der Thiomilchsäure sowie die freien Säuren.

Die schwefelhaltigen, keratinreduzierenden Substanzen werden in den Wellmitteln bevorzugt in Konzentrationen von 0,2 bis 1,5 mol pro 1000g des gesamten Wellmittels eingesetzt.

Der pH-Wert des erfindungsgemäßen Mittels liegt im Bereich von pH 5 bis 12, insbesondere von pH 7 bis 9,5. Der pH-Wert der erfindungsgemäßen Mittel wird bei Verwendung von Sulfit und/oder Disulfit und/oder Hydrogensulft bevorzugt auf einen Wert im Neutralbereich von pH 5 bis 8, bevorzugt von pH 6 bis 7.5 eingestellt.
Zur Einstellung dieses pH-Wertes enthalten die erfindungsgemäßen Wellmittel üblicherweise Alkalisierungsmittel wie Ammoniak, Alkali- und Ammonium-carbonate und -hydrogencarbonate oder organische Amine wie Monoethanolamin. Um eine Schonung der Kopfhaut bei guter Wellwirksamkeit des Wellmittels zu gewährleisten, bleibt der pH-Wert des erfindungsgemäßen Mittels während der Anwendung konstant. Konstant bedeutet im Sinne der Erfindung, dass sich der pH-Wert des anwendungsbereiten Mittels um nicht mehr als 0,5 pH-Einheiten, insbesondere nicht mehr als 0,2 pH-Einheiten, ändert. Somit ist das anwendungsbereite Mittel bevorzugt frei von Verbindungen zu formulieren, welche durch eine im anwendungsbereiten Mittel stattfindende alkalische Hydrolyse eine messbare dynamische pH-Wert Senkung verursachen. Diese Verbindungen sind beispielsweise Diethyloxalat, Galactonsäure-1,4-lacton, Ribonsäure-1,4-lacton, Mannonsäure-1,4-lacton, Gulonsäure-1,4-lacton, Glucuronsäure-6,3-lacton, Gluconsäure-1,5-lacton, sonstige Carbonsäureester in Kombination mit esterspaltenden Enzymen.

Die Verbindungen (I) werden bevorzugt in Mengen von 0,1 bis 5 Gew.-%, insbesondere von 0,3 bis 3 Gew.-%, jeweils bezogen auf das gesamte Wellmittel, eingesetzt.

Die keratinreduzierenden Verbindungen und die Verbindungen (I) werden bevorzugt in einem Gewichtsverhältnis von 130 zu 1 bis 1 zu 2,5, besonders bevorzugt von 37 zu 1 bis 1 zu 1, in die erfindungsgemäßen Mittel eingearbeitet.

Weiterhin können die erfindungsgemäßen Mittel wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, ß-Hydroxyethyl- und ß-Aminoethyl-Gruppen. Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-lsopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Die Aminosäuren können sowohl als freie Aminosäure als auch als Salze, z. B. als Hydrochloride, eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.

Bezüglich näherer Informationen zu solchen wellkraftverstärkenden Komponenten wird auf die Druckschriften DE-OS 44 36 065 und EP-B1-363 057 verwiesen, auf deren Inhalt ausdrücklich Bezug genommen wird.

Die wellkraftverstärkenden Verbindungen können in den erfindungsgemäßen Mitteln in Mengen von 0,5 bis 5 Gew.-%, bezogen auf das gesamte Mittel, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Die erfindungsgemäßen Mittel können zusätzlich mindestens eine Ölkomponente enthalten.

Erfindungsgemäß geeignete Ölkomponenten sind prinzipiell alle Öle und Fettstoffe sowie deren Mischungen mit festen Paraffinen und Wachsen. Bevorzugt sind solche Ölkomponenten, deren Löslichkeit in Wasser bei 20 °C kleiner 1 Gew.-%, insbesondere kleiner als 0,1 Gew.-% beträgt. Der Schmelzpunkt der einzelnen Öl- oder Fettkomponenten liegt bevorzugt unterhalb von etwa 40 °C. Ölkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Aprikosenkernöl, Avokadoöl, Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

Eine weitere, besonders bevorzugte Gruppe erfindungsgemäß einsetzbarer Ölkomponenten sind flüssige Paraffinöle und synthetische Kohlenwasserstoffe sowie Din-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Glycerin, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol^{®} SN), Stearinsäure-2-ethylhexylester (Cetiol^{®} 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl-glycerinmonostearat.

Erfindungsgemäß bevorzugt verwendbare Ölkomponenten sind schließlich auch Silikonöle, insbesondere Dialkyl- und Alkylarylsiloxane, wie beispielsweise Dimethylpolysiloxan und Methylphenylpolysiloxan, sowie deren alkoxylierte und quaternierte Analoga sowie cyclische Siloxane. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80). Bevorzugt im Sinne der Erfindung können Silikonöle mit einer kinematischen Viskosität bis zu 50.000 cSt gemessen bei 25 °C sein. Ganz besonders bevorzugt sind Silikonöle mit kinematischen Viskositäten bis zu 10.000 cSt gemessen bei 25 °C. Die Bestimmung der Viskositäten erfolgt dabei nach der Kugelfallmethode entsprechend der Methode "british standard 188". Vergleichbare Werte werden mit zum "british standard 188" analogen Prüfvorschriften der Hersteller erhalten, beispielsweise der "CTM 0577" der Dow Corning Corporation.

In einer besonderen Ausführungsform werden als Ölkomponente insbesondere cyclische Siloxane wie beispielsweise die Produkte Dow Corning^{®} 344, Dow Corning^{®} 345, Dow Corning^{®} 244, Dow Corning^{®} 245 oder Dow Corning^{®} 246 mit kinematischen Viskositäten von bis zu 10.000 cSt bei 25 °C bestimmt entsprechend den Angaben des Herstellers eingesetzt.

Erfindungsgemäß einsetzbare Ölkomponenten sind schließlich auch Dialkylcarbonate, wie sie in der DE-OS 197 101 54, auf die ausdrücklich Bezug genommen wird, ausführlich beschrieben werden. Dioctylcarbonate, insbesondere das Di-2-ethylhexylcarbonat, sind bevorzugte Ölkomponenten in Rahmen der vorliegenden Erfindung.

Weiterhin können die erfindungsgemäßen Mittel zusätzlich nur begrenzt mit Wasser mischbare Alkohole enthalten.

Unter "mit Wasser begrenzt mischbar" werden solche Alkohole verstanden, die in Wasser bei 20 °C zu nicht mehr als 10 Gew.-%, bezogen auf die Wassermasse, löslich sind.

In vielen Fällen haben sich Triole und insbesondere Diole als erfindungsgemäß besonders geeignet erwiesen. Erfindungsgemäß einsetzbar sind Alkohole mit 4 bis 20, insbesondere 4 bis 10, Kohlenstoffatomen. Die erfindungsgemäß verwendeten Alkohole können gesättigt oder ungesättigt und linear, verzweigt oder cyclisch sein. Einsetzbar im Sinne der Erfindung sind beispielsweise Butanol-1, Cyclohexanol, Pentanol-1, Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Erfindungsgemäß bevorzugt als Alkohole sind 2-Ethyl-hexandiol-1,3, Butanol-1, Cyclohexanol, Pentanol-1 und 1,2-Butandiol. Insbesondere 2-Ethyl-hexandiol-1,3, aber auch Butanol-1 und Cyclohexanol sind besonders bevorzugt.

In einer weiteren Ausführungsform können in den erfindungsgemäßen Mitteln Emulgatoren verwendet werden. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,1 bis 3 Gew.-%, bezogen auf die jeweilige gesamte Zusammensetzung.

Bevorzugt können die erfindungsgemäßen Mittel mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10-15 können erfindungsgemäß besonders bevorzugt sein.

Das erfindungsgemäße Mittel liegt bevorzugt als wässrige Zusammensetzung vor. Eine wäßrige Zusammensetzung im Sinne der Erfindung enthält mindestens 50 Gew.% Wasser bezogen auf das Gewicht der gesamten Zusammensetzung. Diese wäßrige Zusammensetzung kann in verschiedenen Formen, beispielsweise als Lotion, Öl-in-Wasser-Emulsion oder Wasser-in-Öl-Emulsion, vorliegen.

Die erfindungsgemäße Lehre umfaßt jedoch auch solche Ausführungsformen des erfindungsgemäßen Mittels, bei denen ein mehrphasiges Mittel vorliegt, welches aus zwei oder mehr getrennt konfektionierten Ausgangszubereitungen erst unmittelbar vor der Anwendung hergestellt werden kann. Diese Ausführungsform kann bei inkompatiblen Bestandteilen bevorzugt sein. Es hat sich überraschenderweise gezeigt, daß auf diese Weise formulierte Wellmittel bei gleicher Menge der jeweiligen keratinreduzierenden Komponenten einen deutlich erhöhten Welleffekt ergeben. Folglich kann auf diese Weise die mit einem erfindungsgemäß formuliertem Mittel erzielte Welleistung unter deutlicher Verringerung des Anteils der keratinreduzierenden Substanz, und somit unter zusätzlicher Schonung von Haar und Kopfhaut, erreicht werden.

Weiterhin hat sich gezeigt, daß durch Formulierung der Mittel als Zwei- und Mehrphasensystem das Problem der Parfümierung deutlich verringert werden kann. Aufgrund der von den meisten Anwendern nicht tolerierten Duftnote der in den erfindungsgemäßen Mitteln enthaltenen Bestandteile (keratinreduzierende Thio-Verbindungen, ggf. Alkalien wie Ammoniak oder Alkanolamine) ist eine Parfümierung jedoch praktisch unerläßlich. Problematisch ist, daß die überwiegende Zahl der Parfümkomponenten in solchen Mitteln nicht lagerstabil ist. Somit ist die Auswahl der Duftnoten für diese Mittel stark eingeschränkt. Es hat sich nun ebenfalls überraschenderweise gezeigt, daß bei Verwendung der Zwei- oder Mehrphasensysteme eine Vielzahl weiterer Parfümkomponenten in diese Mittel lagerstabil eingearbeitet werden kann.

Erfindungsgemäß einsetzbare Zwei- und Mehrphasensysteme sind Systeme, bei denen mindestens zwei separate, kontinuierliche Phasen vorliegen. Beispiele für solche Systeme sind Zubereitungen, die folgende Phasen aufweisen:
- eine wäßrige Phase und eine nichtwäßrige Phase, die separat voneinander vorliegen
- eine wäßrige Phase und zwei nichtwäßrige, miteinander nicht mischbare Phasen, die jeweils separat vorliegen
- eine Öl-in-Wasser-Emulsion und eine davon separiert vorliegende nichtwäßrige Phase
- eine Wasser-in-Öl-Emulsion und eine davon separiert vorliegende wäßrige Phase.

Keine Zwei-Phasensysteme im Sinne der vorliegenden Erfindung sind Systeme, bei denen nur eine kontinuierliche Phase vorliegt, wie z. B. reine Öl-in-Wasser- oder Wasser-in-Öl-Emulsionen.

Weiterhin hat es sich als vorteilhaft erwiesen, wenn die erfindungsgemäßen Mittel einen pflegenden Wirkstoff, ausgewählt aus Proteinhydrolysaten und deren Derivaten, enthalten.

Geeignete Proteinhydrolysate sind insbesondere Elastin-, Kollagen-, Keratin-, Milcheiweiß-, Seidenprotein-, Sojaprotein-, Mandelprotein-, Erbsenprotein-, Kartoffelprotein-, Haferprotein-, Maisprotein- und Weizenproteinhydrolysate. Dabei können Produkte auf pflanzlicher Basis erfindungsgemäß bevorzugt sein.

Geeignete Derivate sind insbesondere quaternisierte Proteinhydrolysate. Beispiele für diese Verbindungsklasse sind die unter den Bezeichnungen Lamequat^{®}L(CTFA-Bezeichnung: Lauryldimonium Hydroxypropylamino Hydrolyzed Animal Protein; Grünau), Croquat^{®}WKP und Gluadin^{®}WQ auf dem Markt befindlichen Produkte. Das letztgenannte Produkt, das auf pflanzlicher Basis beruht, kann bevorzugt sein.

Die Protein-Derivate sind in den erfindungsgemäßen Mitteln bevorzugt in Mengen von 0,1 bis 10 Gew.-%, bezogen auf die Gesamtmenge des Mittels enthalten. Mengen von 0,1 bis 5 Gew.-% sind bevorzugt.

Bevorzugt enthalten die erfindungsgemäßen Mittel weiterhin mindestens einen konditionierenden Wirkstoff.

Als konditionierende Wirkstoffe kommen bevorzugt kationische Polymere in Betracht. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- Polysiloxane mit quaternären Gruppen,
- Polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammo-niumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Vinylimidazoliniummethochlorid-Copolymere, wie sie unter der Bezeichnung Luviquat^{®} angeboten werden,
- quaternierter Polyvinylalkohol
sowie die unter den Bezeichnungen
- Polyquaternium 2,
- Polyquaternium 17,
- Polyquaternium 18 und
- Polyquaternium 27
bekannten Polymeren mit quartären Stickstoffatomen in der Polymerhauptkette.

Geeignet als konditionierende Wirkstoffe sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat^{®}2001 N im Handel erhältlich sind sowie das Handelsprodukt Merquat^{®} 280, sind erfindungsgemäß besonders bevorzugte Ampho-Polymere.

Die kationischen oder amphoteren Polymere sind in den erfindungsgemäßen Zubereitungen bevorzugt in Mengen von 0,1 bis 5 Gew.-%, bezogen auf die gesamte Zubereitung, enthalten.

Als konditionierende Wirkstoffe eignen sich weiterhin Silikon-Gums, wie z.B. das Handelsprodukt Fancorsil^{®} LIM-1, sowie anionische Silikone, wie beispielsweise das Produkt Dow Corning^{®}1784.

Beispiele für die in den erfindungsgemäßen Mitteln als konditionierende Wirkstoffe verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, sogenannte "Esterquats", wie beispielsweise die unter den Warenzeichen Dehyquart^{®} und Stepantex^{®} vertriebenen Methyl-hydroxyalkyldialkoyloxyalkyl-ammoniummethosulfate, eingesetzt werden.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Weiterhin kann es bevorzugt sein, die einzelnen Phasen mit Farbstoffen anzufärben, um ein besonders gutes optisches Erscheinungsbild des Mittels zu erzielen. Diese Farbstoffe sind bevorzugt nur in der wäßrigen oder nur in mindestens einer nichtwäßrigen Phase in einer Menge löslich, die eine entsprechende Einfärbung für den Betrachter sichtbar erscheinen läßt. Es ist auch möglich, sowohl die nichtwäßrige als auch die wäßrige Phase mit verschiedenen Farbstoffen, bevorzugt in verschiedenen Farben, einzufärben. Das alleinige Anfärben einer nichtwäßrigen Phase ist jedoch bevorzugt.

Weitere übliche Bestandteile für die erfindungsgemäßen Mittel sind:
- anionische Tenside wie beispielsweise Seifen, Alkylsulfate und Alkylpolyglykolethersulfate, Salze von Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure Alkylglykosiden oder Alkoholen, welche Anlagerungsprodukte von etwa 2-15 Molekülen Ethylenoxid und/oder Propylenoxid an Fettalkohole mit 8 bis 22 C-Atomen darstellen.
- zwitterionische Tenside wie beispielsweise Betaine und 2-Alkyl-3-carboxymethyl-3-hydroxyethyl-imidazoline.
- ampholytische Tenside, wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren.
- nichtionische Tenside wie beispielsweise Anlagerungsprodukte von 2 bis 30 Mol Ethylenoxid und/oder 0 bis 5 Mol Propylenoxid an lineare Fettalkohole mit 15 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.
- nichtionische Polymere wie beispielsweise Vinylpyrrolidon/Vinylacrylat-Copolymere, Polyvinylpyrrolidon und VinylpyrrolidonNinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden, Polyoxycarbonsäuren, wie Polyketo- und Polyaldehydocarbonsäuren und deren Salze,
   sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- organische Verdickungsmittel wie Agar-Agar, Guar-Gum, Alginate, Celluloseether, wie Methyl- und Methylhydroxypropylcellulose, Gelatine, Pektine und/oder Xanthan-Gum. Ethoxilierte Fettalkohole, insbesondere solche mit eingeschränkter Homologenverteilung, wie sie beispielsweise als Handelsprodukt unter der Bezeichnung Arlypon^{®}F (Henkel) auf dem Markt sind, alkoxylierte Methylglucosidester, wie das Handelsprodukt Glucamate^{®} DOE 120 (Amerchol), und ethoxylierte Propylenglykolester, wie das Handelsprodukt Antil^{®} 141 (Goldschmidt), können bevorzugte organische Verdickungsmittel sein.
- Strukturanten wie Glucose und Maleinsäure,
- haarkonditionierende Verbindungen wie Phospholipide, beispielsweise Sojalecithin, Ei-Lecithin und Kephaline,
- Parfümöle,
- Lösungsvermittler, wie Ethanol, Isopropanol, Ethylenglykol, Propylenglykol, Glycerin, Diethylenglykol und ethoxylierte Triglyceride sowie Fettalkoholethoxylate und deren Derivate,
- Antischuppenwirkstoffe wie Climbazol, Piroctone Olamine und Zink Omadine,
- Wirkstoffe wie Bisabolol, Allantoin, Panthenol, Niacinmid, Tocopherol und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe sowie
- Treibmittel wie Propan-Butan-Gemische, N₂O, Dimethylether, CO₂ und Luft.

Bezüglich der weiteren Bestandteile der erfindungsgemäßen Mittel und deren übliche Einsatzmengen wird ausdrücklich auf die bekannten Monographien, z. B. Umbach, Kosmetik, 2. Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Ein dritter Gegenstand der Erfindung ist ein Verfahren zur dauerhaften Umformung keratinhaltiger Fasern, insbesondere menschlicher Haare, in welchem
(i) die Fasern unter Zuhilfenahme von Verformungshilfsmitteln nach, vor oder während des Schritts (ii) verformt werden,
(ii) ein Mittel des zweiten Erfindungsgegenstandes auf die Fasern aufgetragen wird,
(iii) die Fasern nach einer Einwirkzeit Z1, gespült und gegebenenfalls getrocknet werden,
(iv) anschließend ein Fixiermittel, enthaltend mindestens ein Oxidationsmittel, auf die Fasern aufgetragen und nach einer Einwirkzeit Z2 wieder abgespült wird.

Verformungshilfsmittel im Sinne des erfindungsgemäßen Verfahrens können
- z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickler, im Rahmen eines Dauerwellverfahrens für einen längeren Zeitraum an der Faser befestigt werden, so ist es zweckmäßig, diese Verformungshilfsmittel vor Schritt (iii) oder nach Schritt (iv) zu entfernen. Es kann in diesem Zusammenhang vorteilhaft sein, die Verformungshilfsmittel während des Schritts (iv) im Haar zu belassen, sie danach zu entfernen und danach Schritt (iv) als sogenannten Nachfixierschritt (v) zu wiederholen.

In einer bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Unter einer mechanischen Glättung wird erfindungsgemäß eine Streckung der krausen Faser entlang ihrer längsten räumlichen Ausdehnung verstanden.

Die Einwirkzeit Z1 beträgt bevorzugt 5-60 Minuten, besonders bevorzugt 10-30 Minuten. Die Einwirkzeit Z2 beträgt bevorzugt 1-30 Minuten, besonders bevorzugt 5-20 Minuten.

Zwingender Bestandteil der im erfindungsgemäßen Verfahren verwendeten Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 15 Gew.-%, gebrauchsfertig in der Regel etwa 0,5 bis 3 Gew.-%, H₂O₂ enthalten, liegt bevorzugt bei 2 bis 6, insbesondere 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere H₂O₂, enthalten. Die Fixiermittel enthalten bevorzugt mindestens 50 Gew.-% Wasser.

Die folgenden Beispiele sollen die Erfindung näher erläutern.

### Beispiele

Es wurden nach bekannten Verfahren die Rezepturen der untenstehenden Beispiele 1 bis 6 hergestellt. Jedes Beispiel umfasst eine erfindungsgemäße Zubereitung, welche mit einem W gekennzeichnet ist, und eine Vergleichszusammensetzung, die mit einem V gekennzeichnet wird. Jede Beispielformulierung wurde an 10 Probanden im Halbseitentest unter Ausführung des folgenden Dauerwellverfahrens getestet:
Das gesamte Kopfhaar wird mit Wasser angefeuchtet und mit einem Handtuch frottiert.

Das Kopfhaar wird mit einem Kamm in eine linke und eine rechte Hälfte gescheitelt. Anschließend wird das Haar auf Wickler aufgewickelt. Die linke Hälfte wird mit 30 mL der erfindungsgemäßen Zubereitung W behandelt und die rechte Halbseite mit 30 mL der korrespondierenden Vergleichsrezeptur V des jeweiligen Beispiels.

Nach einer Einwirkzeit von 20 Minuten wird das Haar mit Wasser gespült und 25 mL eines Fixiermittels gemäß nachfolgender Tabelle 1 auf den gesamten Haarbereich aufgetragen.

Nach einer Einwirkzeit von 10 Minuten werden die Wickler entfernt und das Haar gründlich gespült.

Anschließend wird das erzielte Wellergebnis und der Zustand der Kopfhaut visuell bewertet und Daten über das Gefühl der behandelten Kopfhaut durch Befragung des Probenden erhoben.

In allen Beispielformulierungen wurden folgende Rohstoffe verwendet:
¹ wässrige Lösung mit 60% Aktivsubstanz
² wässrige Lösung mit 60% Aktivsubstanz
³ wässrige, 25%ige Lösung
⁴ Poly(dimethyldiallylammoniumchlorid) (INCI-Bezeichnung: Polyquaternium-6) (Nalco)
⁵ Kokosfettalkohol alkoxyliert mit 10 Einheiten Ethylenoxid, überwiegend C₁₂- bis C₁₄-Fettalkohole (INCI-Bezeichnung: Coceth-10) (Clariant)
⁶ ethoxyliertes Rizinusöl (INCI-Bezeichnung: PEG-40 Hydrogenated Castor Oil) (BASF)
⁷ Polydimethylsiloxan mit 3-(2-Aminoethylamino)propylgruppen (INCI-Bezeichnung: Amodimethicone) (Wacker)
⁸ Decamethylcyclopentasiloxan (INCI-Bezeichnung: Cyclomethicone) (Dow Corning)
⁹ Hydroxyethylcellulose (INCI-Bezeichnung: Hydroxyethylcellulose) (Hercules)
¹⁰ 1-Hydroxyethan-1,1-diphosphonsäure, wässrige Lösung mit ca. 60% Aktivsubstanz (INCI-Bezeichnung: Etidronic Acid) (Solutia)
" Natriumsalz des N-Cocoylweizenproteinkondensats, wässrige Zubereitung mit 24-27% Aktivsubstanz (INCI-Bezeichnung: Sodium Cocoyl Hydrolyzed Wheat Protein) (Zschimmer & Schwarz)
¹² 3-Dodecyidimethylammonium-2-hydroxypropyl-Weizenproteinhydrolysat (INCI-Bezeichnung: Laurdimonium Hydroxypropyl Hydrolyzed Wheat Protein) (Cognis)

### Beispiel 1: Wellmittel für schwer wellbares Haar

| Inhaltsstoffe | W1 | V1 |
|---|---|---|
| Ammoniumthioglykolat ¹ (60 % Aktivsubstanz) | 17,00 g | 17,00 g |
| Ammoniumthiolactat (60 % TMS) ² | 1,30 g | 1,30 g |
| Ammoniumbicarbonat | 4,50 g | 4,50 g |
| Ammoniak 25 % ³ | 2,50 g | 2,50 g |
| Harnstoff | 2,10 g | 2,10 g |
| Merquat® 100 ⁴ | 0,60 g | 0,60 g |
| Genapol® C 100 ⁵ | 1,00 g | - |
| Cremophor® CO 40 ⁶ | 1,00 g | 1,00 g |
| Parfüm | | |
| Wasser | ad 100 g | ad 100 g |

Beide Rezepturen ergaben ein akzeptables Wellergebnis. Bei der Verwendung der Zusammensetzung V1 wurden vereinzelt Rötungen des behandelten Kopfhautbereichs beobachtet. Der Proband fühlte diese Art von Hautirritation als Missempfinden auf den entsprechenden Hautpartien. Die Rezeptur W1 lieferte eine verbesserte Verträglichkeit bei reduzierten Hautrötungen und -irritationen.

### Beispiel 2: Wellmittel für gefärbtes Haar

| Inhaltsstoffe | W2 | V2 |
|---|---|---|
| Ammoniumthioglykolat ¹ | 12,00 g | 12,00 g |
| Ammoniumbicarbonat | 0,80 g | 0,80 g |
| Ammoniak 25 % ³ | 0,50 g | 0,50 g |
| Merquat® 100 ⁴ | 0,60 g | 0,60 g |
| Genapol® C 100 ⁵ | 1,00 g | - |
| Cremophor® CO 40 ⁶ | 1,00 g | 1,00 g |
| Parfüm | | |
| Wasser | ad 100 g | ad 100 g |

Beide Rezepturen ergaben ein akzeptables Wellergebnis. Die Zusammensetzung V2 verursachte vereinzelt Rötungen des damit behandelten Kopfhautbereichs. Der Proband fühlte diese Art von Hautirritation als Missempfinden auf den entsprechenden Hautpartien. Die Rezeptur W2 lieferte eine verbesserte Verträglichkeit bei reduzierten Hautrötungen und -irritationen.

### Beispiel 3: Wellmittel mit Wärmeentwicklung

| Inhaltsstoffe | W3 | V3 |
|---|---|---|
| Komponente A: | | |
| Ammoniumthioglykolat ¹ | 25,00 g | 25,00 g |
| Ammoniumbicarbonat | 7,0 g | 7,00 g |
| Ammoniak 25 % ³ | 2,9 g | 2,90 g |
| Merquat® 100 ⁴ | 0,6 g | 0,60 g |
| Genapol® C 100 ⁵ | 1,0 g | - |
| Cremophor® CO 40 ⁶ | 1,0 g | 1,00 g |
| Parfüm | | |
| Wasser | ad 100 g | ad 100 g |
| | | |

| Komponente B: | | |
|---|---|---|
| Ammoniak | 0,50 g | 0,50 g |
| Dipicolinsäure | 0,50 g | 0,50 g |
| Wasserstoffperoxid 50 % | 6,00 g | 6,00 g |
| Wasser | ad 100 g | ad 100 g |

Die Komponenten A und B des Beispiels 3 wurden jeweils im Gewichtsverhältnis 4 : 1 unter Erhalt des jeweiligen anwendungsbereiten Wellmittels gemischt. Durch die Wärmeentwicklung wurden verbesserte Wellergebisse erzielt. Bei der Verwendung der Zusammensetzung V3 wurden vereinzelt Rötungen des behandelten Kopfhautbereichs beobachtet. Der Proband fühlte diese Art von Hautirritation als Missempfinden auf den entsprechenden Hautpartien. Die Rezeptur W3 lieferte eine verbesserte Verträglichkeit bei reduzierten Hautrötungen und -irritationen.

### Beispiel 4: Pflegendes Wellmittel für gefärbtes Haar

| Inhaltsstoffe | W4 | V4 |
|---|---|---|
| Phase 1: | | |
| Harnstoff | 2,00 g | 2,00 g |
| 2-ethyl-1,3-Hexandiol | 0,30 g | 0,30 g |
| Ammoniumthioglykolat ¹ | 12,00 g | 12,00 g |
| Ammoniak 25 % ³ | 3,50 g | 3,50 g |
| Ammoniumbicarbonat | 5,00 g | 5,00 g |
| Cremophor® CO 40 ⁶ | 1,50 g | 1,50 g |
| Genapol® C 100 ⁵ | 1,00 g | - |
| Merquat® 100 ⁴ | 2,00 g | 2,00 g |
| Wasser | ad 100 g | ad 100 g |
| | | |

| Phase 2: | | |
|---|---|---|
| Wacker Belsil ADM 652 ⁷ | 7,00 g | 7,00 g |
| Dow Corning 345 Fluid ⁸ | 88,00 g | 88,00 g |
| Parfüm | 5,00 g | 5,00 g |

Die Phasen 1 und 2 des Beispiels 4 werden jeweils im Gewichtsverhältnis 10:1 unter Erhalt des jeweiligen anwendungsbereiten Wellmittels gemischt. Beide Rezepturen ergaben ein akzeptables Wellergebnis bei guter Pflege. Auf dem mit der Zusammensetzung V4 behandelten Kopfhautbereich wurden vereinzelt Rötungen beobachtet. Der Proband fühlte diese Art von Hautirritation als Missempfinden auf den entsprechenden Hautpartien. Die Rezeptur W4 lieferte eine verbesserte Verträglichkeit bei reduzierten Hautrötungen und -irritationen.

### Beispiel 5: Wellmittel für normales Haar

| Inhaltsstoffe | W5 | V5 |
|---|---|---|
| Wellotion A: | | |
| Ammoniumbicarbonat | 3,00 g | 3,00 g |
| Ammoniak 25 % ³ | 1,20 g | 1,20 g |
| Cremophor® CO 40 ⁶ | 1,00 g | 1,00 g |
| Genapol® C 100 ⁵ | 1,00g | - |
| Natrosol® 250 HR ⁹ | 0,80 g | 0,80 g |
| Merquat® 100 ⁴ | 2,00 g | 2,00 g |
| Parfüm | | |
| Wasser | ad 100 g | ad 100 g |
| | | |

| Aktivator B: | | |
|---|---|---|
| Ammoniumthioglykolat ¹ | 70,00 g | 70,00 g |
| Wasser | ad 100g | ad 100 g |

Die Wellotion A und der Aktivator B des Beispiels 5 werden jeweils im Gewichtsverhältnis 4 : 1 unter Erhalt des anwendungsbereiten Wellmittels gemischt. Beide Rezepturen ergaben ein akzeptables Wellergebnis. Bei der Verwendung der Zusammensetzung V5 wurden vereinzelt Rötungen des behandelten Kopfhautbereichs beobachtet. Der Proband fühlt diese Art von Hautirritation als Missempfinden auf den entsprechenden Hautpartien. Die Rezeptur W5 liefert eine verbesserte Verträglichkeit bei reduzierten Hautrötungen und -irritationen.

### Beispiel 6:

| Inhaltsstoffe | W6 | V6 |
|---|---|---|
| Ammoniumthioglykolat ¹ | 17,00 g | 17,00 g |
| Ammoniak 25 % ³ | 1,70 g | 1,70 g |
| Ammoniumbicarbonat | 8,00 g | 8,00 g |
| Cremophor® CO 40 ⁶ | 1,00 g | 1,00 g |
| Genapol® C 100 ⁵ | 1,00 g | - |
| Merquat® 100 ⁴ | 0,10 g | 0,10 g |
| Turpinal® SL ¹⁰ | 0,30 g | 0,30 g |
| Protelan VE/K ¹¹ | 1,00 g | 1,00 g |
| Gluadin® WQ ¹² | 0,10 g | 0,10 g |
| Parfüm | | |
| Wasser | ad 100 g | ad 100 g |

Beide Rezepturen ergaben ein akzeptables Haarumformungsergebnis. Die Zusammensetzung V6 verursachte vereinzelt Rötungen des damit behandelten Kopfhautbereichs. Der Proband fühlte diese Art von Hautirritation als Missempfinden auf den entsprechenden Hautpartien. Die Rezeptur W6 lieferte eine verbesserte Verträglichkeit bei reduzierten Hautrötungen und -irritationen.

## Patentansprüche

1. Verwendung von mit 10 Einheiten Ethylnoxid ethoxylierte Cocasfettalkoholen aus überwiegend (C₁₂- bis C₁₄)- Fettalkoholen zur Verminderung der Hautreizung von schwefelhaltigen, keratinreduzierenden Substanzen.

2. Verwendung nach einem der Ansprüche 1 oder 2, **dadurch gekennzeichnet, dass** die schwefelhaltige, keratinreduzierende Substanz ausgewählt wird, aus der Gruppe, die gebildet wird aus Cysteamin, Cystein, Bunte Salzen und Salzen der schwefligen Säure, Thioglykolsäure, Thiomilchsäure, Thioäpfelsäure, Mercaptoethansulfonsäure sowie den Salzen und Estern der vorgenannten Thiosäuren.

3. Mittel für die dauerhafte Verformung keratinhaltiger Fasern, insbesondere menschlicher Haare, welches einen konstanten pH-Wert aufweist und
(a) mindestens eine schwefelhaltige, keratinreduzierende Substanz sowie
(b) mit 10 Einheiten Ethylenoxid ethoxylierte Cocosfettalkohole aus überwiegend (C₁₂- bis C₁₄) - Fettalkoholen
enthält.

4. Mittel nach Anspruch 3, **dadurch gekennzeichnet, dass** die schwefelhaltige, keratinreduzierende Substanz ausgewählt wird, aus der Gruppe, die gebildet wird aus Cysteamin, Cystein, Bunte Salzen und Salzen der schwefligen Säure, Thioglykolsäure, Thiomilchsäure, Thioäpfelsäure, Mercaptoethansulfonsäure sowie den Salzen und Estern der vorgenannten Thiosäuren.

5. Mittel nach einem der Ansprüche 3 oder 4, **dadurch gekennzeichnet, dass** zusätzlich ein konditionierender Wirkstoff enthalten ist.

6. Verfahren zur dauerhaften Umformung keratinhaltiger Fasern, insbesondere menschlicher Haare, in welchem
(i) die Fasern unter Zuhilfenahme von Verformungshilfsmitteln nach, vor oder während des Schritts (ii) verformt werden,
(ii) ein Mittel nach einem der Ansprüche 3 bis 5 auf die Fasern aufgetragen wird,
(iii) die Fasern nach einer Einwirkzeit Z1, gespült und gegebenenfalls getrocknet werden,
(iv) anschließend ein Fixiermittel, enthaltend mindestens ein Oxidationsmittel, auf die Fasern aufgetragen und nach einer Einwirkzeit Z2 wieder abgespült wird.

## Claims

1. Use of coconut fatty alcohols, predominantly comprising (C₁₂ to C₁₄) fatty alcohols, ethoxylated with 10 units of ethylene oxide for reducing skin irritation caused by sulfur-containing, keratin-reducing substances.

2. Use according to either one of claims 1 or 2, **characterised in that** the sulfur-containing, keratin-reducing substance is selected from the group formed from cysteamine, cysteine, Bunte salts and salts of sulfurous acid, thioglycolic acid, thiolactic acid, thiomalic acid, mercaptoethanesulfonic acid and the salts and esters of the above-stated thioacids.

3. An agent for permanent deformation of keratin-containing fibres, in particular human hair, which has a constant pH and
(a) at least one sulfur-containing, keratin-reducing substance and
(b) coconut fatty alcohols, predominantly comprising (C₁₂ to C₁₄) fatty alcohols, ethoxylated with 10 units of ethylene oxide.

4. An agent according to claim 3, **characterised in that** the sulfur-containing, keratin-reducing substance is selected from the group formed from cysteamine, cysteine, Bunte salts and salts of sulfurous acid, thioglycolic acid, thiolactic acid, thiomalic acid, mercaptoethanesulfonic acid and the salts and esters of the above-stated thioacids.

5. An agent according to either one of claims 3 or 4, **characterised in that** a conditioning active ingredient is additionally included.

6. A method for permanent reshaping of keratin-containing fibres, in particular human hair, in which
(i) the fibres are deformed with the assistance of deformation aids before, during or after step (ii),
(ii) an agent according to any one of claims 3 to 5 is applied to the fibres,
(iii) the fibres are rinsed after an exposure time Z1 and optionally dried,
(iv) then a fixing agent containing at least one oxidant is applied to the fibres and rinsed off again after an exposure time Z2.

## Revendications

1. Utilisation d'alcools gras de coco éthoxylés avec 10 unités d'oxyde d'éthylène, constitués d'alcools gras principalement en C₁₂-C₁₄ pour diminuer l'irritation de la peau de substances contenant du soufre, réduisant la kératine.

2. Utilisation selon l'une quelconque des revendications 1 ou 2, **caractérisée en ce que** la substance contenant du soufre, réduisant la kératine, est choisie dans le groupe formé par la cystéamine, la cystéine, les sels de Bunte et les sels de l'acide sulfureux, l'acide thioglycolique, l'acide thiolactique, l'acide thiomalique, l'acide mercaptoéthanesulfonique ainsi que les sels et les esters des thioacides susmentionnés.

3. Agent pour la mise en forme permanente de fibres kératiniques, en particulier de cheveux humais, qui présente un pH constant et qui contient
(a) au moins une substance contenant du soufre, réduisant la kératine, ainsi que
(b) des alcools gras de coco éthoxylés avec 10 unités d'oxyde d'éthylène, constitués par des alcools gras principalement en C₁₂-C₁₄.

4. Agent selon la revendication 3, **caractérisé en ce que** la substance contenant du soufre, réduisant la kératine, est choisie dans le groupe formé par la cystéamine, la cystéine, les sels de Bunte et les sels de l'acide sulfureux, l'acide thioglycolique, l'acide thiolactique, l'acide thiomalique, l'acide mercaptoéthanesulfonique ainsi que les sels et les esters des thioacides susmentionnés.

5. Agent selon l'une quelconque des revendications 3 ou 4, **caractérisé en ce qu'**une substance active de conditionnement est en outre contenue.

6. Procédé pour la mise en forme permanente de fibres kératiniques, en particulier de cheveux humains, dans lequel
(i) les fibres sont mises en forme à l'aide d'adjuvants de mise en forme, après, avant ou pendant l'étape (ii),
(ii) un agent selon l'une quelconque des revendications 3 à 5 est appliqué sur les fibres,
(iii) les fibres sont rincées après un temps d'action Z1 et le cas échéant séchées,
(iv) un fixateur, contenant au moins un oxydant, est ensuite appliqué sur les cheveux et à nouveau éliminé par rinçage après un temps d'action Z2.
